(19) **Europäisches Patentamt**
**European Patent Office**
**Office européen des brevets**

(11) **EP 3 096 804 B1**

(12) **EUROPEAN PATENT SPECIFICATION**

(45) Date of publication and mention
of the grant of the patent:
**04.12.2019 Bulletin 2019/49**

(51) Int Cl.:
*A61L 27/26* (2006.01)          *A61L 27/38* (2006.01)
*A61L 27/54* (2006.01)          *A61L 27/52* (2006.01)

(21) Application number: **15707784.3**

(22) Date of filing: **19.01.2015**

(86) International application number:
**PCT/IB2015/050389**

(87) International publication number:
**WO 2015/107502 (23.07.2015 Gazette 2015/29)**

(54) **METHOD OF MAKING A HYDROGEL, HYDROGEL AND FORMULATION FOR CARRIERS AND/OR SUBSTITUTE OF CONNECTIVE TISSUES OBTAINED USING SUCH METHOD**

VERFAHREN ZUR HERSTELLUNG EINES HYDROGELS, HYDROGEL UND FORMULIERUNG FÜR TRÄGER UND/ODER ERSATZ VON MIT DIESEM VERFAHREN HERGESTELLTEN BINDEGEWEBEN

PROCÉDÉ DE FABRICATION D'UN HYDROGEL, HYDROGEL ET FORMULATION POUR SUPPORT ET/OU SUBSTITUT DE TISSUS CONJONCTIFS OBTENUS PAR UN TEL PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.01.2014 IT VI20140011**

(43) Date of publication of application:
**30.11.2016 Bulletin 2016/48**

(73) Proprietor: **Bioteck S.p.A.**
**36057 Arcugnano (VI) (IT)**

(72) Inventor: **FIORINI, Mauro**
**I-40057 Granarolo Dell'Emilia (BO) (IT)**

(74) Representative: **Maroscia, Antonio**
**Maroscia & Associati Srl**
**Piazza del Castello, 26**
**36100 Vicenza (IT)**

(56) References cited:
**WO-A2-2005/086694      US-A- 5 540 033**
**US-A1- 2008 139 694**

**Description**

Field of the Invention

The present invention generally finds application in the field of biomaterials for implantation in the human body and namely relates to a method of making a hydrogel.

The invention also relates to a hydrogel and a formulation comprising such hydrogel, which are designed for use as carriers and/or substitute of connective tissues of autologous, homologous, heterologous, synthetic origin.

Background art

Biomaterials are known to be transplanted in the human body for regeneration/substitute of of certain tissue types after traumas or certain pathological conditions.

As a rule, such biomaterials may be of synthetic origin or derive from biological materials and may be transplanted in the body by means of surgery or injection in the region concerned by the disease or the trauma.

Namely, the main purpose of these biomaterials is to replace the extracellular matrix of connective tissues, and promote regeneration of the damaged tissue by supporting and inducing cell proliferation, possibly in response to certain mechanical stimuli from within or outside the body.

These biomaterials include, amongst others, hydrogels based on polymers that can be solubilized and/or hydrated in aqueous solutions, and are polymerized and sterilized by γ or β radiation. Water-soluble polymers may have different molecular weights according to their degree of polymerization, i.e. the number of basic units that compose their chemical structure.

Particularly, the final viscosity of the aqueous solution, and hence that of the desired gel, is given by the molecular weight of water-soluble polymers in direct proportion, according to the Mark-Houwink equation, which is as follows:

$$[\eta]=KM^a$$

where $[\eta]$ is the viscosity of the solution, K is the direct proportionality constant, M is the molecular weight of tehe polymer, a is a parameter associated with the polymer-solvent interaction.

Nevertheless, the main drawback of these materials is the degree of polymerization obtained upon irradiation is not easily controllable.

The difficulty in controlling polymerization causes difficulty in regulating the viscosity of the biomaterial, and hence reduces the ability to predict its physico/chemical behavior versus the tissue in which it is implanted.

Generally, these hydrogels have lower mechanical properties than the extracellular matrix, which limits proliferation of implanted cells and thus causes only partial tissue regeneration.

In an attempt to obviate these drawbacks, hydrogels have been developed which comprise, in addition to the above mentioned water-soluble polymers, additional components designed to limit the polymerization process after irradiation.

US5540033 discloses a PEO (polyethylene oxide)-based hydrogel comprising a polymerization inhibitor agent selected from the group comprising ascorbic acid and its derivatives.

Particularly, ascorbic acid allows sequestration of free radicals formed upon irradiation, thereby delaying polymerization reactions of polyethylene oxide (PEO) molecules.

Nevertheless, a first drawback of these materials is that the use of ascorbic acid and/or its derivatives in combination with PEO only delays polymerization, and hence viscosity is not constantly regulated.

As a result, this hydrogel tends to change its mechanical properties with time upon the action of biological tissues or the adhesion and proliferation of cells and other biological material on its surface.

A further drawback is that the use of these particular types of components provides a hydrogel having some consistency, but unsuitable for injection using syringes or similar instruments.

Finally, a serious drawback is that the viscosity of the material before and after irradiation may change considerably, and this will require repeated actions on the hydrogel composition before implantation.

WO2015/086694 discloses a method of use of an occlusive biomedical device with a hydrogel comprising cellulose and ascorbic acid. PEG is used to shrink the hydrogel but using no radiation

US2008/0139694 discloses a hydrogel comprising amongst others cellulose and PEG/PEO in combination. Polymerization is achieved without use of radiation.

Disclosure of the invention

The object of the present invention is to overcome the above drawbacks, by providing a method of making a hydrogel, as well as a hydrogel and a formulation for carriers and/or substitute of connective tissues that are highly efficient and relatively cost-effective.

A further object of the present invention is to provide a hydrogel and a formulation for carriers and/or substitute of connective tissues that allow viscosity to remain substantially constant over time.

Another object of the present invention is to provide a hydrogel and a formulation for carriers and/or substitute of connective tissues whose mechanical and chemical properties are suitable to enhance cell proliferation.

Also, a further object of the present invention is to provide a hydrogel and a formulation for carriers and/or substitute of connective tissues whose mechanical and chemical properties are substantially similar to those of the extracellular matrix of the tissues to be reintegrated.

Another object of the present invention is to provide a method of making a hydrogel, a hydrogel and a formulation for carriers and/or substitute of connective tissues that can provide implantable products having differentiated consistencies.

These and other objects, as more clearly explained hereafter, are fulfilled by a method of making a hydrogel as defined in claim 1.

In a further aspect, the invention relates to a hydrogel and a formulation for carriers and/or substitute of connective tissues as defined in claims 10 and 12 respectively.

With these characteristics, a controllable-viscosity hydrogel may be obtained, which may act as a support for the growth of substitute of cells for damaged biological tissues, while mimicking the mechanical properties of the extracellular matrix.

Advantageous embodiments of the invention are as defined in the dependent claims.

Brief description of the drawings

Further features and advantages of the invention will be more readily apparent upon reading of the detailed description of a non-limiting embodiment of a method of making hydrogels, a hydrogel and a formulation for carriers and/or substitute of connective tissues, which are shown as a non limiting example with the help of the annexed figures, in which:

FIGURES 1 to 4 show diagrams of the consistency and/or viscoelasticity curves of a first hydrogel sample with a water-soluble polymer having a first molecular weight;

FIGURES 5 and 6 show diagrams of the consistency and/or viscoelasticity curves of a second hydrogel sample with the same water-soluble polymer as that of Figures 1 to 4, with a second molecular weight, different from the first;

FIGURES 7 to 10 show diagrams of the consistency and/or viscoelasticity curves of a third hydrogel sample with a second water-soluble polymer having a molecular weight different from the first two.

Detailed description of a few preferred embodiments

The present invention addresses a method of making a hydrogel designed for use as a carrier and/or substitute of connective tissue.

A hydrogel obtained using the method of the invention may be advantageously directly implanted *in situ* in the human body, due to its high biocompatibility and its virtual non-toxicity to the biological tissues to be regenerated.

Particularly, the method may provide a hydrogel that can mimic the structure of the extracellular matrix for connective tissue regeneration.

In its basic form, the method comprises a step of providing a predetermined amount of an aqueous solution, a step of adding a predetermined amount of a polymerization regulator agent in the aqueous solution, for regulating the polymerization of a first electromagnetic radiation-polymerizable water-soluble polymer and a step of adding, hydrating and/or solubilizing a predetermined amount of the first water-soluble polymer in the aqueous solution.

Preferably, the aqueous solution may be selected from the group comprising PBS or other buffered salines.

The use of PBS will allow the operating pH to be maintained at a value substantially close to the physiological value of 7.4, such that the hydrogel also has a pH compatible with that of the body.

Conveniently, the amount of the first water-soluble polymer is selected according to the volume of the aqueous solution and the final amount of hydrogel to be obtained, to prevent saturation of the solution, and formation of precipitates that might alter the final physico/chemical properties of the hydrogel.

Furthermore, the step of adding, hydrating and/or solubilizing the first water-soluble polymer in the aqueous solution may be speeded up using well-known processes, such as magnetic stirring, mechanical stirring or heating of the aqueous solution.

The steps of adding the regulator agent and the first water-soluble polymer provide a homogeneous aqueous solution mixture.

This solution may have a predetermined concentration of the first water-soluble polymer, designed to provide homogenous solubilization and prevent the occurrence of precipitates or flocs.

Furthermore, the hydration and/or solubilization step may be carried out through repeated additions of partial amounts of the first water-soluble polymer, in view of speeding up the process and providing a homogeneous mixture.

The step of adding the first water-soluble polymer is followed by a step of applying an electromagnetic radiation of predetermined frequency to the mixture, for achieving polymerization of the first polymer with a predetermined viscosity, and for obtaining a sterile hydrogel.

Advantageously, the electromagnetic radiation allows a radical rearrangement reaction to be activated on the molecules of the first water-soluble polymer, to promote reaction among the molecules and hence their polymerization.

Preferably, the electromagnetic radiation applied to the mixture may have a frequency that falls in the range of ionizing radiations, preferably close to the frequency spectrum of β rays.

The electromagnetic radiation of type β may be applied to the mixture for the latter to have an average radiation-absorbed dose of 25 KGy.

Furthermore, the electromagnetic radiation of type β will also promote sterilization of the mixture, to eliminate any risk of bacterial or viral infection upon implantation of the hydrogel.

Advantageously, the step of applying the electromagnetic radiation may be preceded by a step of combining the mixture with biological material of the substitute of connective tissue type, such that the latter may be sterilized upon irradiation.

According to a peculiar aspect of the invention, a second radiation-polymerizable water-soluble polymer, which is different from the first and non-reactive to the regulator, is added to the aqueous solution before application of the electromagnetic radiation to obtain a sterile hydrogel.

Particularly, the amount of the regulator agent relative to the first water-soluble polymer and the amount of the second polymer are selected to allow control the final consistency and viscosity of the sterile hydrogel.

Conveniently, long-lasting control of the viscosity of the hydrogel is essential to obtain a biomaterial whose chemical, physical and mechanical properties are substantially identical to those of the extracellular matrix to be replaced.

In the case of water-soluble polymers, the consistency and viscolaesticity values of the hydrogel obtained at the end of the process are related to the degree of polymerization of the polymer, i.e. the number of monomers that compose the polymer chain, and to the molecular weight of the polymer.

Particularly, as the number of monomers that form the chains increases, the degree of polymerization also increases, and provides high consistency and viscoelasticity values of the final product.

Advantageously, the regulator agent is adapted to regulate the degree of polymerization of the first water-soluble polymer and hence, indirectly, the final consistency and viscoelasticity of the hydrogel.

Furthermore, the second water-soluble polymer will be selected in view of being fully polymerized upon application of the electromagnetic radiation.

This method, particularly due to the combined effect of the regulator agent and the second water-soluble polymer, will provide a hydrogel having constant controlled consistency and viscoelasticity with time.

A hydrogel obtained using the method of the present invention may be an ideal medium for proliferation of substitute of connective tissue cells that can mimic the mechanical properties of the extracellular matrix.

Conveniently, the regulator agent is selected from the group comprising ascorbic acid and may be adapted to reduce the degree of polymerization of the first water-soluble polymer as its concentration in the mixture increases, as shown by the diagrams of the figures.

As is known in the art, ascorbic acid, or vitamin C, generally acts as an antioxidant, by sequestering the free radicals formed as a result of certain electrochemical reactions.

Advantageously, in the method of the present invention, ascorbic acid sequesters the radical species of the first water-soluble polymer formed upon application of the electromagnetic radiation, thereby fostering limitation of the polymerization process and the final degree of polymerization of the hydrogel.

Thus, if such radical species of the water-soluble polymer were not sequestered by ascorbic acid, they might keep on reacting with one another and continue their polymerization process.

The regulator agent may also be selected from the group comprising carotenoids, lipoic acid, vitamins E, D, B and glutathione, which also have similar antioxidative effects to the radical species.

The concentration of the regulator agent in the mixture is maintained at a value of 100 mM or less.

Particularly, such ascorbic acid concentration will promote post-irradiation protection to the biological material combined with the mixture.

Nevertheless, a regulator concentration of 2 mM was also experimentally found to be sufficient to modulate and control the degree of polymerization of the first water-soluble polymer, and hence the final viscosity of the hydrogel.

The first water-soluble polymer is selected from a first group comprising polyethylene glycol (PEG) to obtain an injectable hydrogel.

The polyethylene glycol (PEG) suitable for preparation of the hydrogel may have a molecular weight ranging from 10 K g/mol to 100 Kg/mol, preferably from 10 Kg/mol to 40 Kg/mol.

Particularly suitable polyethylene glycol (PEG) types may have molecular weights of 10 Kg/mol, 20 Kg/mol and 35 Kg/mol respectively.

The use of these types of polyethylene glycol (PEG), in combination with the regulator agent and the second water-soluble polymer, will provide a hydrogel that may have suitable consistency and viscoelasticity values for an injectable preparation.

Alternatively, the first water-soluble polymer is selected from a second group comprising polyethylene oxide (PEO) to obtain a substantially semisolid hydrogel.

The polyethylene oxide (PEO) suitable for preparation of the hydrogel may have a molecular weight ranging from 100 Kg/mol to 1000 Kg/mol, preferably from 100 Kg/mol to 600 Kg/mol.

Advantageously, suitable polyethylene oxide (PEO) types may have molecular weights of 100 Kg/mol, 200 Kg/mol, 400 Kg/mol and 600 Kg/mol respectively.

The use of these types of polyethylene oxide (PEO), in combination with the regulator agent and the second water-soluble polymer, will provide a hydrogel with higher consistency and viscoelasticity values than the first hydrogel.

The weight percent of the first water-soluble polymer ranges from 1% to 20% based on the total weight of the hydrogel.

Particularly, the weight percent of polyethylene glycol (PEG) polymer in an injectable preparation may range from 5% to 15% based on the total weight of the hydrogel.

Alternatively, the weight percent of polyethylene oxide (PEO) polymer may range from 2% to 10% based on the total weight of the hydrogel.

The second water-soluble polymer is selected from the group comprising cellulose derivatives such as hydroxyethyl cellulose (HEC), hydroxypropyl methylcellulose (HPMC), ethyl cellulose (EC), methyl cellulose (MC) or the like.

The use of cellulose derivatives promotes constant control of hydrogel viscosity, and prevents their solubilization from interfering with the solubilization of the first water-soluble polymer.

Furthermore, polymerization of cellulose monomers, particularly for low-concentration solutions, mainly occurs by an esterification reaction which is not affected by ascorbic acid or its derivatives and other anti-oxidant agents as regulator agents.

For this reason, cellulose polymers are inert to the regulator agent, and their polymerization continues independent of the polymerization of the first water-soluble polymer.

Nevertheless, polymerization of cellulose polymers may be affected and/or limited by intermolecular steric hindrance by the chains of the first water-soluble polymer, whose degree of polymerization is modulated by ascorbic acid and/or its derivatives.

The weight percent of the second water-soluble polymer ranges from 0.1% to 10%, preferably from 0.5% to 5% based on the total weight of the hydrogel.

Of course, the use of cellulose as the second water-soluble polymer shall be intended by way of example and without limitation, as cellulose may be replaced by other water-soluble polymers having equivalent viscoelastic properties.

In a further aspect, the invention relates to a hydrogel designed for use as a carrier and/or a substitute of connective tissue, which comprises an aqueous solution, a first polymer polymerized in an aqueous solution and a predetermined amount of the regulator agent for regulating the degree of polymerization of the first polymer in the aqueous solution.

A peculiar aspect of this hydrogel is that it comprises a predetermined amount of a second water-soluble polymer which is different from the first and is inert to the regulator agent.

Furthermore, the amounts of the regulator agent and the second water-soluble polymer are selected to allow control of the final consistency and viscosity of the sterile hydrogel.

Advantageously, the first water-soluble polymer is selected from the group comprising polyethylene glycol (PEG) or polyethylene oxide (PEO), whereas the regulator agent may be selected from the group comprising ascorbic acid.

Furthermore, the second water-soluble polymer is selected from the group comprising cellulose derivatives such as hydroxyethyl cellulose (HEC), hydroxypropyl methylcellulose (HPMC) or the like.

This hydrogel may have different consistency and viscoelasticity values according to the type of first water-soluble polymer in use and to the amount of regulator agent that has been added during its preparation.

Particularly, a hydrogel comprising polyethylene glycol (PEG) may have low consistency and viscoelasticity, and may be used as a carrier and/or an injectable substitute of connective tissue, whereas a hydrogel comprising polyethylene glycol (PEO) may have relatively high consistency and viscoelasticity and may be adapted for use as a carrier and/or a semisolid pre-formed substitute of connective tissue.

Also, in a further aspect, the invention relates to a formulation designed for use as a carrier for cells and/or connective tissues of autologous, heterologous or synthetic origin and/or bioactive molecules.

Particularly, the cells may be bone and/or cartilage tissue cells of equine origin.

The bioactive molecules may be selected from the group comprising growth factors, drugs or bacteriocins.

It shall be understood that the formulation may comprise cells different from those mentioned above, derived from non-bone tissues of autologous, homologous or synthetic origin.

Two examples of two different hydrogel compositions of the invention are provided below.

EXAMPLE 1

Preparation of a polyethylene glycol (PEG)-based hydrogel and analysis of the consistency and viscoelasticity of the hydrogel

The hydrogel was prepared by first providing a predetermined volume of an aqueous solution, which depends on the final desired amount and weight of the hydrogel.

The aqueous solution preferably consisted of a Phosphate Buffered Saline (PBS) at 1X concentration.

Predetermined amounts of ascorbic acid and/or its derivatives were later solubilized in PBS by magnetic stirring using an ARGOLAB M2-A stirrer in a beaker at 300 rpm for 10 min or to full solubilization of ascorbic acid and/or its derivatives.

Then, the solid components, particularly the water-soluble polymers to be later dissolved in the aqueous solution, were weighed.

Particularly, two different samples were prepared, referred to as LMW 35K and LMW 20K, which comprise two types of polyethylene glycol (PEG) whose molecular weights are 35K g/mol and 20K g/mol respectively. Then, a constant amount of hydroxypropyl methylcellulose (HPMC) was added to both samples.

The weight ratio of polyethylene glycol (PEG) to hydroxypropyl methylcellulose (HPMC) was substantially maintained at a value of 15:1 or less.

Then the two polymers (PEG and HPMC) were added slowly using a spatula.

While polymers were being added, the solution was maintained under stirring in a beaker using a fixed-blade column stirrer (ARGOLAB AM20-D) at 300 rpm. The mixture was stirred for 24 h or to full hydration of the polymers.

Different ascorbic acid concentrations were used for each of the two samples, such that their effect on the final consistency and viscoelasticity of the hydrogel could be assessed.

After solubilization of the polymers, the mixture so obtained was distributed in appropriate plastic or glass containers, or combined with substitute of connective tissues.

Later, the mixture was irradiated with electromagnetic radiation of type $\beta$ to promote ascorbic acid-mediated polymerization of the polyethylene glycol (PEG) and hydroxypropyl methylcellulose (HPMC).

The samples so obtained underwent a test for measuring consistency and viscoelasticity using the Stable Micro Systems TA.XT Plus texture analyzer.

Particularly, the samples were placed in cylindrical plastic glasses and a compression and relaxation test was carried out using an appropriate cylindrical probe P25.

The test consists in causing a probe to slide in the hydrogel to a fixed distance of 10 mm at a constant speed of 0.5 mm/s, and, after 120 seconds waiting time, monitoring relaxation of the sample.

The measure of the compression resistance force when 10 mm have been covered provides quantification of the viscoleasticity of each sample.

The viscosity values of both samples LMW 35K and LMW 20K and their different compositions are set forth in Table and Table II below.

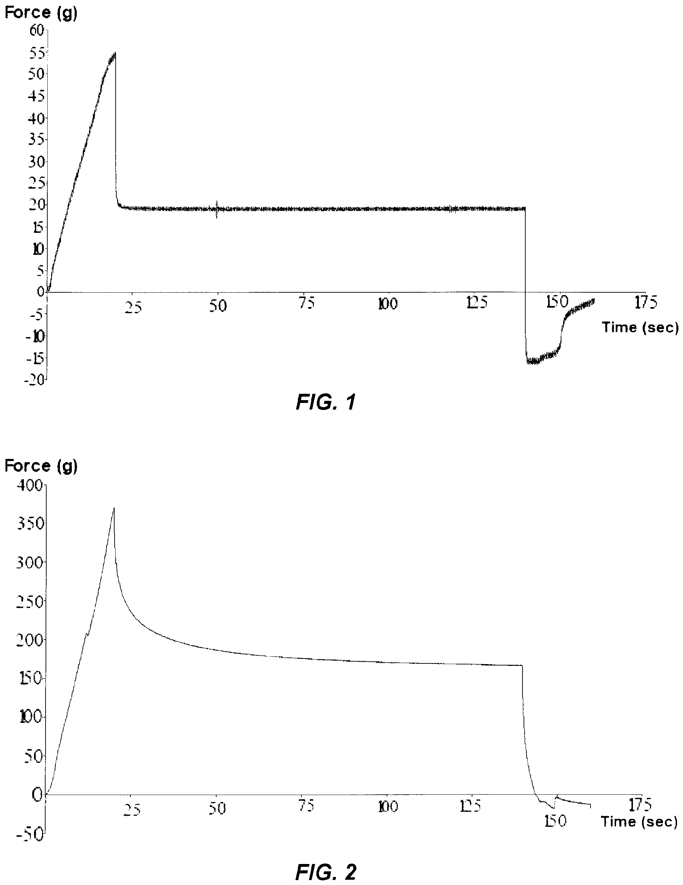

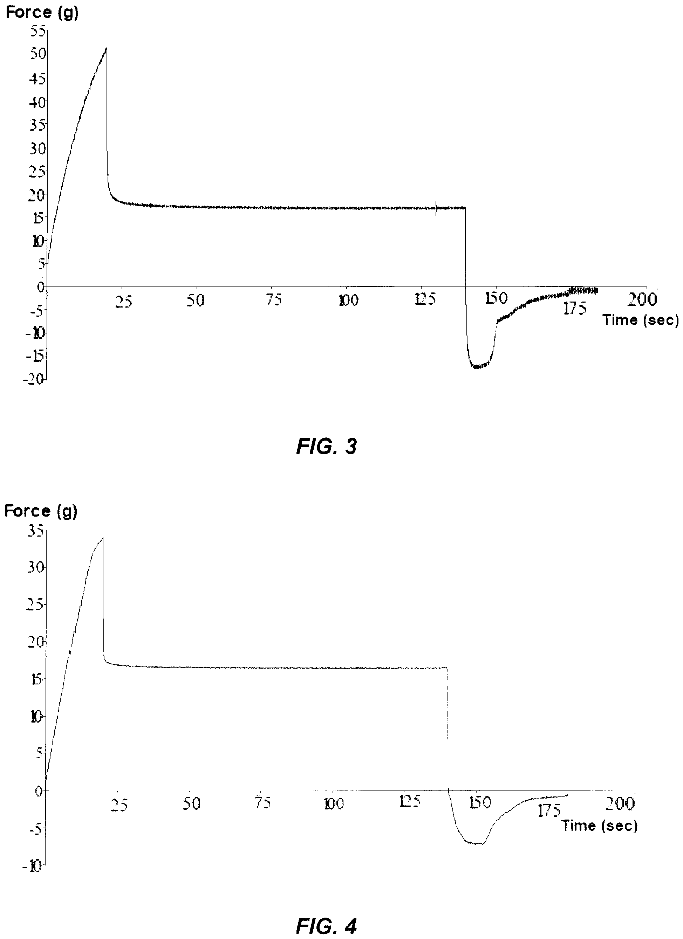

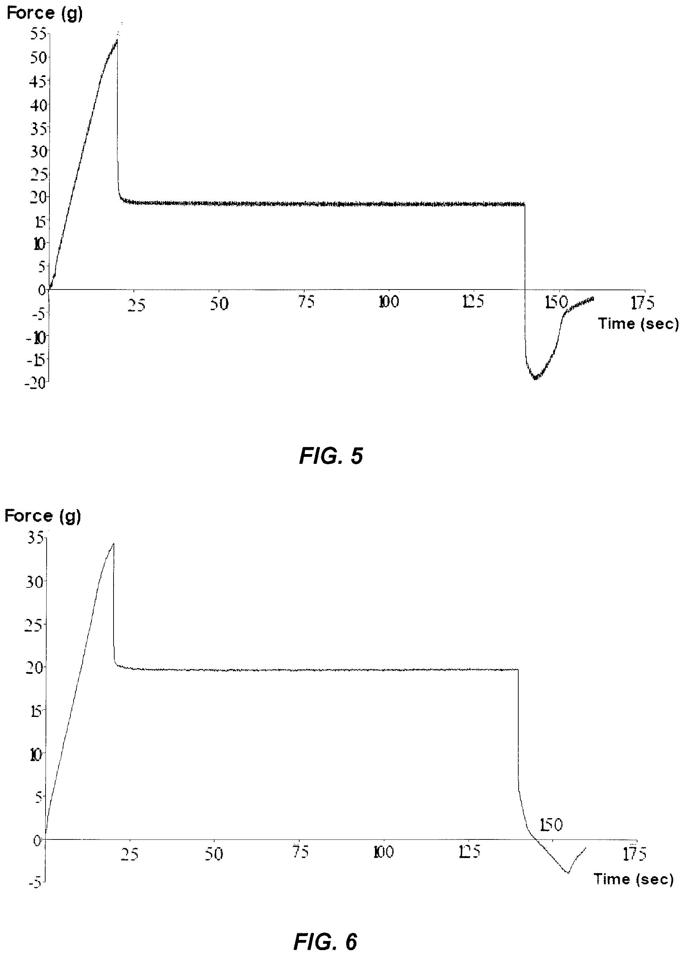

The values of Table I are represented in the charts of FIGS. 1-4, whereas the values of the samples 10NS and 10S of Table II are represented in the charts of FIGS. 5, 6.

**Table I**

| Numerical code | Formulation | Probe | Average force (g) |
|---|---|---|---|
| 21 NS | LMW 35K, w/o Vit. C - NON STERILE | P25 | 35.35 |
| 21 S | LMW 35K w/o Vit. C - STERILE | P25 | 372.46 |
| 22 S | LMW 35K Vit.C 1mM - STERILE | P25 | 34.34 |

(continued)

| Numerical code | Formulation | Probe | Average force (g) |
|---|---|---|---|
| 23 S | LMW 35K Vit.C 2mM - STERILE | P25 | 17.66 |
| Hydrogel LMW 35K (Low Molecular Weight): PEG 35 Kg/mol, HPMC K15M, Sodium Ascorbyl Phosphate (SAP), PBS1X | | | |

**Table II**

| 8 NS | LMW 20K, w/o Vit. C - NON STERILE | P25 | 35.38 |
|---|---|---|---|
| 10 NS | LMW 20K, Vit.C 2mM - NON STERILE | P25 | 35.28 |
| 9S | LMW 20K Vit.C 1mM - STERILE | P25 | 32.41 |
| 10 S | LMW 20K Vit.C 2mM - STERILE | P25 | 14.95 |
| Hydrogel LMW 20K (Low Molecular Weight): PEG 20 Kg/mol, HPMC K15M, Sodium Ascorbyl Phosphate (SAP), PBS1X | | | |

The words "NON STERILE" and "STERILE" designate the samples before and after application of the electromagnetic radiation respectively.

The tables and charts show that the addition of increasing concentrations of ascorbic acid limits the consistency and viscoelasticity of the hydrogel upon application of the electromagnetic radiation.

Without ascorbic acid, viscosity tends to reach very high values, due to the higher degree of polymerization of polyethylene glycol (PEG), and injectability of the hydrogel is not ensured.

Furthermore, it will be appreciated that, for both preparations comprising polyethylene glycol (PEG), viscosity is substantially unchanged before and after application of the electromagnetic radiation due to the effect of ascorbic acid at 1 mM concentration.

EXAMPLE 2

Preparation of a polyethylene oxide (PEO)-based hydrogel and analysis of the consistency and viscoelasticity of the hydrogel

The hydrogel was prepared by first providing a predetermined volume of an aqueous solution, which depends on the final desired amount and weight of the hydrogel.

The aqueous solution preferably consisted of a Phosphate Buffered Saline (PBS) at 1X concentration.

Predetermined amounts of ascorbic acid and/or its derivatives were later solubilized in PBS by magnetic stirring using an ARGOLAB M2-A stirrer in a beaker at 300 rpm for 10 min or to full solubilization of ascorbic acid and/or its derivatives.

Then, the solid components, particularly the water-soluble polymers to be later dissolved in the aqueous solution, were weighed.

Particularly, a sample comprising polyethylene oxide (PEO) was prepared, whose molecular weight was 400 Kg/mol. Then, a constant amount of hydroxypropyl methylcellulose (HPMC) was added to the sample.

The weight ratio of polyethylene oxide (PEO) to hydroxypropyl methylcellulose (HPMC) was substantially maintained at a value of 15:1 or less.

Then the two polymers were added slowly using a spatula.

While polymers were being added, the solution was maintained under stirring in a beaker using a fixed-blade column stirrer (ARGOLAB AM20-D) at 100 rpm.

The mixture was stirred for 24 h or to full hydration of the polymers.

Different ascorbic acid concentrations were used such that their effect on the final consistency and viscoelasticity of the hydrogel could be assessed.

After solubilization of the polymers, the mixture so obtained was distributed in appropriate plastic or glass containers, or combined with substitute of connective tissues.

Later, the mixture was irradiated with electromagnetic radiation of type $\beta$ to promote ascorbic acid-mediated polymerization of the polyethylene oxide (PEO) and hydroxypropyl methylcellulose (HPMC).

The samples so obtained underwent a test for measuring consistency and viscoelasticity using the Stable Micro Systems TA.XT Plus texture analyzer.

Particularly, the samples were placed in cylindrical plastic glasses and a compression and relaxation test was carried out using an appropriate cylindrical probe P25.

The test consists in causing a probe to slide in the hydrogel to a fixed distance of 10 mm at a constant speed of 0.5 mm/s, and, after 120 seconds waiting time, monitoring relaxation of the sample.

The measure of the compression resistance force when 10 mm have been covered provides quantification of the viscoleasticity of each sample.

The viscosity values of the samples and their different compositions are set forth in Table III below.

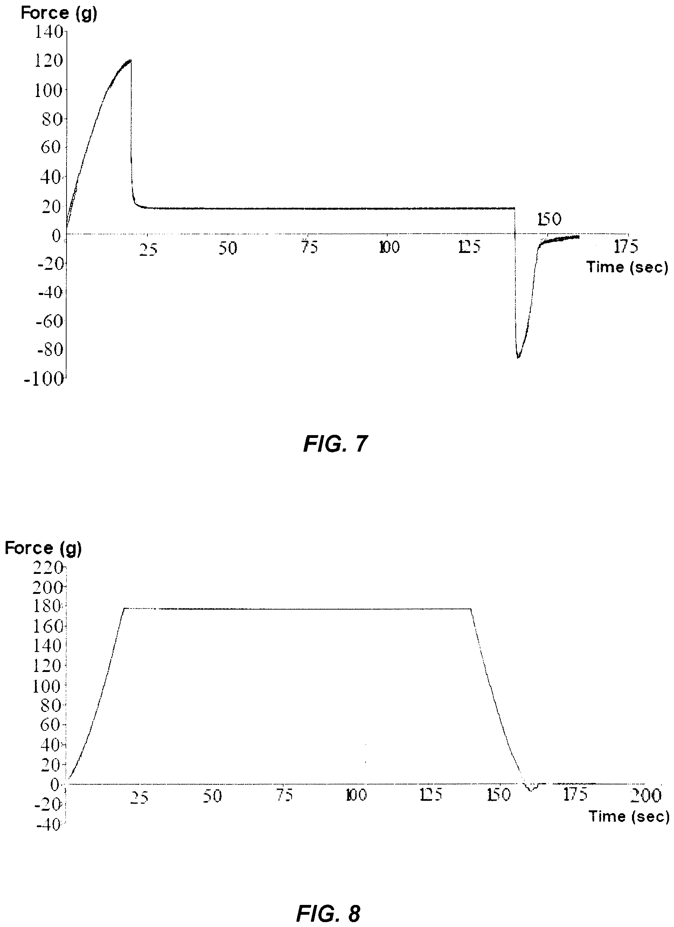

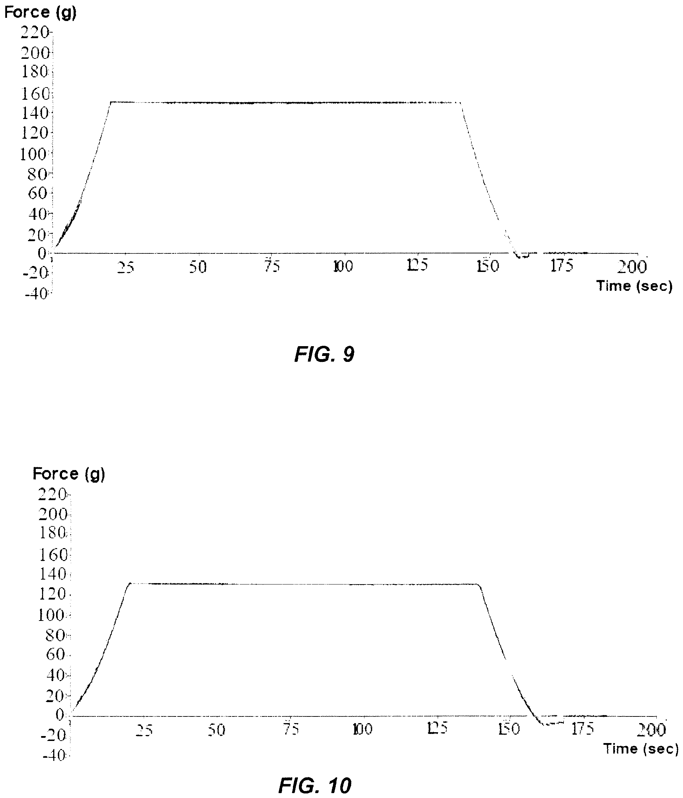

The values of Table III are represented in the charts of FIGS. 7-10.

**Table III**

| Numerical code | Formulation | Probe | Average force (g) |
|---|---|---|---|
| 14 NS | HMW, w/o Vit. C - NON STERILE | P25 | 101.77 |
| 14 S | HMW, w/o Vit. C - STERILE | P40 | 178.19 |
| 15 S | HMW Vit.C 0.5 mM - STERILE | P40 | 150.84 |
| 16 S | HMW Vit.C 2 mM - STERILE | P40 | 132.18 |
| Hydrogel HMW (High Molecular Weight): PEO 400 Kg/mol, HPMC K15M, Sodium Ascorbyl Phosphate (SAP), PBS1X | | | |

The tables and charts show that the addition of increasing concentrations of ascorbic acid limits and progressively reduces the consistency and viscoelasticity of the hydrogel upon application of the electromagnetic radiation.

Without ascorbic acid, viscosity tends to reach very high values, due to the higher degree of polymerization of polyethylene oxide (PEO).

The method, hydrogel and formulation of the invention are susceptible of many changes and variants within the inventive principle disclosed in the annexed claims.

Industrial Applicability

The present invention finds industrial application in the field of medical devices and biomaterials for therapeutic and medical/cosmetic use, and particularly in the field of biomaterials for implantation in the human body as substitute of connective tissues.

**Claims**

1. A method of making a hydrogel for use as a carrier and/or a substitute of connective tissue, comprising the steps of:

   - providing a predetermined amount of an aqueous solution;
   - adding a predetermined amount of a polymerization regulator agent to said aqueous solution, for regulating the polymerization of a first electromagnetic radiation-polymerizable water-soluble polymer;
   - adding, hydrating and/or solubilizing a predetermined amount of said first water-soluble polymer in said aqueous solution, to obtain a mixture;
   - adding, hydrating and/or solubilizing a second electromagnetic radiation-polymerizable water-soluble polymer, different from the first, in said mixture;
   - applying an electromagnetic radiation of predetermined frequency to said mixture, for achieving polymerization of said first polymer and said second polymer with a predetermined viscosity, and for obtaining a sterile hydrogel;

   **characterized in that** said second water-soluble polymer is non-reactive to said regulator agent and has a degree of polymerization that varies according to the intermolecular steric hindrance of the chains of said first polymer, said first water-soluble polymer being selected from a first group comprising polyethylene glycol (PEG) or from a second group comprising polyethylene oxide (PEO), the weight percent of said first water-soluble polymer ranging from 1% to 20% based on the total weight of the hydrogel, said second water-soluble polymer being selected from the group comprising hydroxyethyl cellulose (HEC), hydroxypropyl methylcellulose (HPMC), ethyl cellulose (EC), methyl cellulose (MC), the weight percent of said second water-soluble polymer ranging from 0.1% to 10%, preferably from 0.5% to 5% based on the total weight of the hydrogel, said regulator agent being selected from the group comprising ascorbic acid, carotenoids, lipoic acid, vitamins E, D, B and glutathione, the concentration of said regulator agent in the mixture being 100 mM or less.

2. A method as claimed in claim 1, **characterized in that** said first water-soluble polymer is selected from a first group comprising polyethylene glycol and has a molecular weight ranging from 10 Kg/mol to 100 Kg/mol, preferably from 10 Kg/mol to 40 Kg/mol, for said hydrogel to be of injectable type.

3. A method as claimed in claim 1, **characterized in that** said first water-soluble is selected from a second group comprising polyethylene oxide and has a molecular weight ranging from 100 Kg/mol to 1000 Kg/mol, preferably from 100 Kg/mol to 600 Kg/mol, for said hydrogel to be substantially semisolid and pre-formable.

4. A method as claimed in claim 1, **characterized in that** said regulator agent is selected from the group comprising ascorbic acid, said regulator agent being adapted to reduce the degree of polymerization of said first water-soluble polymer as its concentration in the mixture increases.

5. A method as claimed in claim 1, **characterized in that** said electromagnetic radiation has a frequency that falls in the range of ionizing radiations, and is preferably close to the frequency spectrum of β rays.

6. A hydrogel for use as a carrier and/or a substitute of connective tissue, comprising an aqueous solution, a first water-soluble polymer polymerized in said aqueous solution, a second water-soluble polymer, different from the first, and a predetermined amount of a regulator agent for regulating the degree of polymerization of said first polymer in said aqueous solution, **characterized in that** said first water-soluble polymer is selected to be reactive to said regulator agent and said second water-soluble polymer is selected to be non-reactive to the regulator agent and to have a degree of polymerization influenced by the intermolecular steric hindrance of the chains of said first water-soluble polymer, said first water-soluble polymer being selected from a first group comprising polyethylene glycol (PEG) or from a second group comprising polyethylene oxide (PEO), the weight percent of said first water-soluble polymer ranging from 1% to 20% based on the total weight of the hydrogel, said second water-soluble polymer being selected from the group comprising hydroxyethyl cellulose (HEC), hydroxypropyl methylcellulose (HPMC), ethyl cellulose (EC), methyl cellulose (MC), the weight percent of said first water-soluble polymer ranging from 1% to 20% based on the total weight of the hydrogel, said regulator agent being selected from the group comprising ascorbic acid, carotenoids, lipoic acid, vitamins E, D, B and glutathione, the concentration of said regulator agent being 100 mM or less, the weight percent of said second water-soluble polymer ranges from 0,1% to 10%, preferably from 0.5% to 5% based on the total weight of the hydrogel.

7. A hydrogel as claimed in claim 6, **characterized in that** said second water-soluble polymer is non-reactive to said regulator agent and **in that** said second polymer has a degree of polymerization that varies according to the intermolecular steric hindrance of the chains of said first polymer.

8. A formulation designed for use as a carrier for cells and/or connective tissues of autologous, heterologous or synthetic origin, and/or bioactive molecules, comprising a hydrogel as claimed in one of claims 6 and 7.

**Patentansprüche**

1. Ein Verfahren zur Herstellung eines Hydrogels zur Verwendung als Träger und/oder Ersatz für Bindegewebe, umfassend die Schritte:

- bereitstellen einer vorbestimmten Menge einer wässrigen Lösung;
- zugeben einer vorbestimmten Menge eines Polymerisationsregulierungsmittels zu der wässrigen Lösung, um die Polymerisation eines ersten, durch elektromagnetische Strahlung polymerisierbaren, wasserlöslichen Polymers zu regulieren;
- hinzufügen, hydratisieren und/oder solubilisieren einer vorbestimmten Menge des ersten wasserlöslichen Polymers in der wässrigen Lösung, um eine Mischung zu erhalten;
- hinzufügen, Hydratisieren und/oder solubilisieren eines zweiten, durch elektromagnetische Strahlung polymerisierbaren, wasserlöslichen Polymers, das sich von dem ersten unterscheidet, zu der Mischung;
- anwenden einer elektromagnetischen Strahlung einer vorbestimmten Frequenz auf die Mischung, um eine Polymerisation des ersten Polymers und des zweiten Polymers mit einer vorbestimmten Viskosität zu erreichen und um ein steriles Hydrogel zu erhalten;

**dadurch gekennzeichnet, dass** das zweite wasserlösliche Polymer gegenüber dem Regulatormittel nicht reaktiv ist und einen Polymerisationsgrad aufweist, der gemäß der intermolekularen sterischen Hinderung der Ketten des ersten Polymers variiert, wobei das erste wasserlösliche Polymer entweder aus einem ersten Gruppe, umfassend Polyethylenglykol (PEG), ausgewählt ist, oder aus einer zweiten Gruppe, umfassend Polyethylenoxid (PEO), wobei der Gewichtsanteil des ersten wasserlöslichen Polymers, bezogen auf das Gesamtgewicht des Hydrogels, im Bereich von 1% bis 20% liegt, wobei das zweite wasserlösliche Polymer aus der Gruppe umfassend Hydroxyethylcellulose (HEC), Hydroxypropylmethylcellulose (HPMC), Ethylcellulose (EC), Methylcellulose (MC) ausgewählt ist, wobei der Gewichtsanteil des zweiten wasserlöslichen Polymers, bezogen auf das Gesamtgewicht des Hydrogels, im Bereich von 0,1% bis 10%, vorzugsweise 0,5% bis 5%, liegt, wobei das Regulatormittel aus der Gruppe bestehend aus Ascorbinsäure, Carotinoiden, Liponsäure, Vitaminen E, D, B und Glutathion ausgewählt ist, wobei die Konzentration des Regulatormittels in der Mischung 100 mM oder weniger beträgt.

2. Verfahren nach Anspruch 1, **dadurch gekennzeichnet, dass** das erste wasserlösliche Polymer aus einer ersten Gruppe ausgewählt ist, die Polyethylenglykol umfasst, und ein Molekulargewicht im Bereich von 10 kg/mol bis 100 kg/mol, vorzugsweise von 10 kg/mol bis 40 kg/mol aufweist, damit das Hydrogel vom injizierbaren Typ ist.

3. Verfahren nach Anspruch 1, **dadurch gekennzeichnet, dass** das erste wasserlösliche Produkt aus einer zweiten Gruppe ausgewählt ist, die Polyethylenoxid umfasst, und ein Molekulargewicht im Bereich von 100 kg/mol bis 1000 kg/mol, vorzugsweise von 100 kg/mol bis 600 kg/mol aufweist, damit das Hydrogel im wesentlichen halbfest und vorformbar ist.

4. Verfahren nach Anspruch 1, **dadurch gekennzeichnet, dass** der Regulatormittel aus der Gruppe umfassend Ascorbinsäure ausgewählt ist, wobei das Regulatormittel angepasst ist, um den Polymerisationsgrad des ersten wasserlöslichen Polymers zu verringern, wenn seine Konzentration in der Mischung zunimmt.

5. Verfahren nach Anspruch 1, **dadurch gekennzeichnet, dass** die elektromagnetische Strahlung eine Frequenz aufweist, die in den Bereich ionisierender Strahlungen fällt und vorzugsweise nahe am Frequenzspektrum von β-Strahlen liegt.

6. Ein Hydrogel zur Verwendung als Träger und/oder Ersatz für Bindegewebe, umfassend: eine wässrige Lösung, ein erstes wasserlösliches Polymer, das in der wässrigen Lösung polymerisiert ist, ein zweites wasserlösliches Polymer, das sich von dem ersten unterscheidet, und eine vorbestimmte Menge von ein Regulatormittel zum Regulieren des Polymerisationsgrades des ersten Polymers in der wässrigen Lösung, **dadurch gekennzeichnet, dass** das erste wasserlösliche Polymer so ausgewählt ist, dass es mit dem Regulatormittel reaktiv ist, und das zweite wasserlösliche Polymer so ausgewählt ist, dass es nicht reaktiv auf das Regulierungsmittel ist, und einen Polymerisationsgrad, der durch die intermolekulare sterische Hinderung der Ketten des ersten wasserlöslichen Polymers beeinflusst wird, wobei das erste wasserlösliche Polymer entweder aus einer ersten Gruppe umfassend Polyethylenglykol (PEG), oder aus einer zweiten Gruppe umfassend Polyethylenoxid (PEO) ausgewählt ist, wobei der Gewichtsanteil des ersten wasserlöslichen Polymers, bezogen auf das Gesamtgewicht des Hydrogels, im Bereich von 1% bis 20% liegt, wobei das zweite wasserlösliche Polymer aus der Gruppe umfassend: Hydroxyethylcellulose (HEC), Hydroxypropylmethylcellulose (HPMC), Ethylcellulose (EC), Methylcellulose (MC) ausgewählt ist, wobei der Gewichtsanteil des ersten wasserlöslichen Polymers, bezogen auf das Gesamtgewicht des Hydrogels, im Bereich von 1% bis 20% liegt, wobei das Regulatormittel aus der Gruppe bestehend aus Ascorbinsäure, Carotinoiden, Liponsäure, Vitaminen E, D, B und Glutathion ausgewählt ist, wobei die Konzentration des Regulatormittels 100 mM oder weniger beträgt und der Gewichtsanteil des zweiten wasserlöslichen Polymers, bezogen auf das Gesamtgewicht des Hydrogels, liegt im Bereich von 0,1% bis 10%, vorzugsweise von 0,5% bis 5%.

7. Hydrogel nach Anspruch 6, **dadurch gekennzeichnet, dass** das zweite wasserlösliche Polymer gegenüber dem Regulatormittel nicht reaktiv ist und dass das zweite Polymer einen Polymerisationsgrad aufweist, der gemäß der intermolekularen sterischen Hinderung der Ketten des ersten Polymers variiert.

8. Eine Formulierung zur Verwendung als Träger für Zellen und/oder Bindegewebe autologen, heterologen oder synthetischen Ursprungs und/oder bioaktive Moleküle, umfassend ein Hydrogel nach einem der Ansprüche 6 und 7.

**Revendications**

1. Un procédé de fabrication d'un hydrogel destiné à être utilisé comme support et/ou substitut de tissu conjonctif, comprenant les étapes consistant à:

   - fournir une quantité prédéterminée d'une solution aqueuse;
   - ajouter une quantité prédéterminée d'un agent régulateur de polymérisation à ladite solution aqueuse, pour réguler la polymérisation d'un premier polymère soluble dans l'eau et polymérisable par rayonnement électromagnétique;
   - ajouter, hydrater et/ou solubiliser une quantité prédéterminée dudit premier polymère soluble dans l'eau dans ladite solution aqueuse, pour obtenir un mélange;
   - ajouter, hydrater et/ou solubiliser un deuxième polymère hydrosoluble polymérisable par rayonnement électromagnétique, différent du premier, dans ledit mélange;
   - appliquer un rayonnement électromagnétique de fréquence prédéterminée audit mélange, pour réaliser une polymérisation de viscosité prédéterminée dudit premier polymère et dudit second polymère, et pour obtenir un hydrogel stérile;

   **caractérisé en ce que** ledit second polymère hydrosoluble est non réactif audit agent régulateur et a un degré de polymérisation qui varie en fonction de l'encombrement stérique intermoléculaire des chaînes dudit premier polymère, ledit premier polymère hydrosoluble étant choisi parmi un premier groupe comprenant du polyéthylène glycol (PEG) ou d'un second groupe comprenant du polyoxyde d'éthylène (PEO), le pourcentage en poids dudit premier polymère hydrosoluble étant compris entre 1% et 20% sur la base du poids total de l'hydrogel, ledit second polymère hydrosoluble étant choisi dans le groupe comprenant l'hydroxyéthylcellulose (HEC), l'hydroxypropylméthylcellulose (HPMC), l'éthylcellulose (EC), la méthylcellulose (MC), le pourcentage en poids dudit second polymère hydrosoluble allant de 0,1% à 10%, de préférence de 0,5% à 5% sur la base du poids total de l'hydrogel, ledit agent régulateur étant choisi dans le groupe comprenant l'acide ascorbique, les caroténoïdes, l'acide lipoïque, les vitamines E, D, B et le glutathion, la concentration dudit agent régulateur dans le mélange étant égal ou inférieur à 100 mM.

2. Procédé selon la revendication 1, **caractérisé en ce que** ledit premier polymère hydrosoluble est choisi dans un premier groupe comprenant du polyéthylène glycol et a un poids moléculaire allant de 10 kg/mol à 100 kg/mol, de préférence de 10 kg/mol à 40 kg/mol pour que ledit hydrogel soit de type injectable.

3. Procédé selon la revendication 1, **caractérisé en ce que** ledit premier hydrosoluble est choisi dans un second groupe comprenant du polyoxyde d'éthylène et a un poids moléculaire compris entre 100 kg/mol et 1000 kg/mol, de préférence entre 100 kg/mol et 600 kg/mol, pour que ledit hydrogel soit sensiblement semi-solide et préformable.

4. Procédé selon la revendication 1, **caractérisé en ce que** ledit agent régulateur est choisi dans le groupe comprenant l'acide ascorbique, ledit agent régulateur étant adapté pour réduire le degré de polymérisation dudit premier polymère soluble dans l'eau à mesure que sa concentration dans le mélange augmente.

5. Procédé selon la revendication 1, **caractérisé en ce que** ledit rayonnement électromagnétique a une fréquence qui tombe dans la plage des rayonnements ionisants et est de préférence proche du spectre de fréquence des rayons β.

6. Un hydrogel destiné à être utilisé comme support et / ou substitut de tissu conjonctif, comprenant une solution aqueuse, un premier polymère soluble dans l'eau polymérisé dans ladite solution aqueuse, un second polymère soluble dans l'eau, différent du premier, et une quantité prédéterminée de un agent régulateur pour réguler le degré de polymérisation dudit premier polymère dans ladite solution aqueuse, **caractérisé en ce que** ledit premier polymère hydrosoluble est choisi pour être réactif audit agent régulateur et ledit deuxième polymère hydrosoluble est choisi pour être non réactif audit agent régulateur et avoir un degré de polymérisation influencé par l'encombrement stérique intermoléculaire des chaînes dudit premier polymère hydrosoluble, ledit premier polymère hydrosoluble étant choisi dans un premier groupe comprenant le polyéthylène glycol (PEG) ou dans un deuxième groupe comprenant de l'oxyde de polyéthylène (PEO), le pourcentage en poids dudit premier polymère hydrosoluble allant de 1 % à 20% par rapport au poids total de l'hydrogel, ledit second polymère hydrosoluble étant choisi dans le groupe comprenant l'hydroxyéthylcellulose (HEC), l'hydroxypropylméthylcellulose (HPMC), l'éthylcellulose (EC), la méthylcellulose (MC), le pourcentage en poids dudit premier polymère hydrosoluble allant de 1% à 20% sur la base du poids total de l'hydrogel, ledit agent régulateur étant choisi dans le groupe comprenant l'acide ascorbique, les caroténoïdes, l'acide lipoïque, les vitamines E, D, B et le glutathion, la concentration dudit agent régulateur étant de 100 mM ou moins, le pourcentage en poids dudit second polymère soluble dans l'eau va de 0,1% à 10%, de préférence de 0,5% à 5% sur la base du poids total de l'hydrogel.

7. Hydrogel selon la revendication 6, **caractérisé en ce que** ledit deuxième polymère hydrosoluble est non réactif vis-à-vis dudit agent régulateur et **en ce que** ledit deuxième polymère a un degré de polymérisation qui varie en fonction de l'encombrement stérique intermoléculaire des chaînes dudit premier polymère.

8. Une formulation conçue pour être utilisée comme support pour cellules et/ou tissus conjonctifs d'origine autologue, hétérologue ou synthétique, et/ou molécules bioactives, comprenant un hydrogel selon l'une des revendications 6 et 7.

**FIG. 1**

**FIG. 2**

**FIG. 3**

**FIG. 4**

**FIG. 5**

**FIG. 6**

**FIG. 7**

**FIG. 8**

**FIG. 9**

**FIG. 10**

**REFERENCES CITED IN THE DESCRIPTION**

**Patent documents cited in the description**

- US 5540033 A
- WO 2015086694 A
- US 20080139694 A